# EUROPEAN PATENT SPECIFICATION

(11) **EP 0 706 509 B1**
(45) Date of publication and mention of the grant of the patent: **05.01.2000**
(21) Application number: 95917976.3
(22) Date of filing: 27.04.1995
(51) Int. Cl.: C07C 235/08, A61K 7/48, A61K 7/06

(54) **SHORT CHAIN 2-HYDROXYCARBOXYLIC ACID-BASED DERIVATIVES OF CERAMIDES**
KURZKETTIGE 2-HYDROXYCARBONSÄUREDERIVATE VON CERAMIDEN
DERIVES DE CERAMIDES A BASE D'ACIDE 2-HYDROXYCARBOXYLIQUE A CHAINE COURTE

(30) Priority: 27.04.1994 EP 94201156
(43) Date of publication of application: 17.04.1996
(73) Proprietor: DSM N.V., 6411 TE Heerlen (NL)
(72) Inventor: LAMBERS, Johannes, Wilhelmus, Jacobus, NL-2641 LB Pijnacker (NL); KOGER, Hein, Simon, NL-2064 XR Spaarndam (NL)
(74) Representative: Visser-Luirink, Gesina, Dr.
(86) International application number: EP9501611
(87) International publication number: WO9529151

(56) References cited:
- EP-A- 0 373 038
- EP-A- 0 482 860
- EP-A- 0 495 624
- CHEMICAL ABSTRACTS, vol. 112, no. 7, 12 February 1990, Columbus, Ohio, US; abstract no. 56573n, page 840 ;column 2 ; & JP,A,01 093 562 (SHIONOGI AND CO., LTD.) 12 April 1989
- BIOCHIMICA ET BIOPHYSICA ACTA, vol.573, 1979, NORTH-HOLLAND pages 73 - 82 RANGA R. VUNNAM ET AL. 'short chain ceramides as substrates for glucocerebroside synthetase'
- DATABASE WPI Week 8838, Derwent Publications Ltd., London, GB; AN 88-266512 & JP,A,63 192 703 (KAO CORP) 10 August 1988

## Description

The present invention relates to new compounds namely active ceramide derivatives. Specifically, the invention relates to 2(alpha)-hydroxy carboxylic acid-based ceramide derivatives. The present invention describes a method for obtaining these compounds. The invention also relates to the topical use of these compounds.

### Background of the invention

The stratum corneum is composed of terminally differentiated keratinocytes, called corneocytes, and lipids, mainly localised in the intercellular spaces in bilayer structures; this arrangement is known as the "bricks and mortar" model (Schurer, N.Y. and Elias, P.M. in "The biochemistry and function of stratum corneum lipids", Adv. Lip. Research, (1991), volume 24, pp. 27-57, Academic Press, San Diego). The stratum corneum is therefore considered to be a two-compartment system, the lipid phase being the only continuous system (Elias, P.M., J. Contr. Rel. (1991), 15, pp. 199-208). The lamellar bilayers are stabilized in an aqueous environment by van der Waals interactions and hydrogen bonds, i.e. hydrophilic aqueous layers are present in between the lipid bilayers (Rehfeld et al., J. Invest. Dermatol. (1988), 91, 499-505).

It has been found that 2-hydroxycarboxylic acids (AHA's), when applied topically to the skin, diminish corneocyte cohesion in the stratum corneum, thus causing exfoliation (synonyms: desquamation, peeling) of the corneocytes, resulting in a smoother, glossy and softer skin. Furthermore, AHA's and related compounds are reported to alleviate signs of skin, nail and hair changes associated with intrinsic and/or extrinsic aging. AHA's are also known to enhance cell renewal and collagen synthesis (Berardesca, E. and Distante F., in: Proceedings of 10^{th} Symposium of the Belgian Association of Dermato-Cosmetic Sciences, pp. 11-23, 1994; Smith, W.P. et al., Cosm. and Toilet. (1994), 109, 41-48).

A disadvantage of the use of AHA's is that relatively high levels of these compounds are required in compositions for topical application (from 2-10%). It is known that AHA's can occasionally irritate or sting a person's skin. This necessitated the development of for example a progressive four-step system wherein the amount of AHA is increased gradually to enable the skin to adapt to increasing amounts of AHA.

It has also been described that AHA's do not display strong water binding capacity (Berardesca and Distante, op. cit.).

Ceramides constitute 20-40% of total skin lipids and are present within the intercellular lipid lamellae of the stratum corneum. They are supposed to have an essential role in structuring and maintaining the water impermeability barrier of the skin.

In order to be effective when applied topically, ceramides (and in general sphingolipids) must be able to penetrate the stratum corneum in order to reach the lipid lamellae of the impermeability barrier. It has been shown (Potts, R.O. and Guy, R.H., in Dermal and Transdermal Drug Delivery, APV-paperback Band 31, Gurny, R. and Teubner, A. eds., 1993) that a positive correlation exists between the permeability coefficient (Kₚ₎ and the hydrophobicity of a compound (the octanol/water partition coefficient, K_{oct}). The natural ceramides from the stratum corneum are lipophilic compounds which do not have an optimal balance between water and fat solubility to enable a good penetration into the skin when externally applied.

The present invention discloses 2(alpha)-hydroxy carboxylic acid-based ceramide derivatives. The ceramide derivatives of the present invention consist of a sphingoid base selected from the group consisting of sphingosines and phytosphingosines in an amide linkage to the following 2-hydroxycarboxylic compounds:
1. 2-hydroxyethanoic acid (glycolic acid)
2. 2-hydroxypropanoic acid (lactic acid)
3. 2-methyl 2-hydroxypropanoic acid (methyllactic acid)
4. 2-hydroxybutanoic acid
5. 2-hydroxypentanoic acid
6. 2-hydroxyhexanoic acid
7. 2-hydroxyheptanoic acid
8. 2-hydroxyoctanoic acid (alpha-hydroxycaprylic acid)
9. 2-hydroxynonanoic acid
10. 2-hydroxydecanoic acid
11. 2-hydroxyundecanoic acid
12. 2-hydroxydodecanoic acid
13. 2-hydroxytetradecanoic acid (alpha-hydroxylauric acid), with the proviso that said ceramide derivative is not hydroxyacetylsphingosine, 2-hydroxybutyroylsphingosine or 2-hydroxydecanoylsphinganine.

The invention further provides a method for preparing these compounds.

In another aspect of the invention, the compounds are used in the preparation of cosmetic and/or pharmaceutical compositions.

Compositions containing the compounds of the invention are suitable for topical use. Specifically, the compositions containing the compounds of the present invention can be used to improve the structure and condition of the skin.

### Detailed description of the invention

The present invention relates to new compounds, namely active ceramide derivatives. Specifically, the invention discloses 2(alpha)-hydroxy carboxylic acid-based ceramide derivatives.

Ceramides form the largest polar lipid class of the epidermal lipids. These ceramides form a structurally heterogenous group of sphingolipids, containing sphingoid bases, in amide linkage with alkanoic acids (both hydroxy- and non-hydroxy-).

The ceramide derivatives of the present invention consist of a sphingoid base selected from the group consisting of sphingosines and phytosphingosines in an amide linkage with the following 2-hydroxycarboxylic compounds:
1. 2-hydroxyethanoic acid (glycolic acid)
2. 2-hydroxypropanoic acid (lactic acid)
3. 2-methyl 2-hydroxypropanoic acid (methyllactic acid)
4. 2-hydroxybutanoic acid
5. 2-hydroxypentanoic acid
6. 2-hydroxyhexanoic acid
7. 2-hydroxyheptanoic acid
8. 2-hydroxyoctanoic acid (alpha-hydroxycaprylic acid)
9. 2-hydroxynonanoic acid
10. 2-hydroxydecanoic acid
11. 2-hydroxyundecanoic acid
12. 2-hydroxydodecanoic acid
13. 2-hydroxytetradecanoic acid (alpha-hydroxylauric acid), with the proviso that said ceramide derivative is not hydroxyacetylsphingosine, 2-hydroxybutyroylsphingosine or 2-hydroxydecanoylsphinganine.

By coupling AHA's to a sphingoid base, ceramide derivatives are formed. These rather lipophilic ceramide derivatives will penetrate easier into the skin than the hydrophilic AHA's. Consequently, the concentration of AHA's required for topical application can be much lower when contained in a ceramide backbone than when applied as a free acid in a cosmetic formulation.

In addition, the integration of AHA's within a ceramide structure has the advantage that the resulting compounds have a lower acidity and, consequently, a lower irritation potential than the corresponding free AHA's. Therefore, relatively high concentrations of the AHA-ceramide derivatives of the present invention can be used without causing irritation and inflammation of the skin.

It should further be noted that three classes of ceramides which are present in the mammalian skin, ceramide 4, ceramide 5 and ceramide 6 II (there are seven different ceramide classes in total, Wertz, P.W. et al. (1985), J. Inv. Derm. 84, 410-412), actually are a combination of a sphingoid base and a alpha-hydroxycarboxylic acid. However, these physiologically occurring AHA's range from 16-30 carbon atoms; species below 16 carbon atoms are not found. The present invention specifically relates to the use of ceramide derivatives containing short-chain alpha-hydroxy acids, i.e. C2-C14 acids.

The present invention further provides a method for preparing these C2-C14 alpha-hydroxy carboxylic acid ceramide derivatives. The 2(alpha)-hydroxy carboxylic acid ceramide derivatives of the present invention are prepared by the following reaction steps:
- a carboxylic acid with the desired chain length is brominated on the 2-position,
- the 2-bromocarboxylic acid is converted into a 2-hydroxy acid,
- the 2-hydroxy acid is acylated,
- the 2-acyloxycarboxylic acid is converted into a mixed anhydride with an alkyl sulfonyl chloride or an alkylaryl sulfonyl chloride, by carrying out the reaction in an organic solvent in the presence of a base,
- the N-2-acyloxyacylamido alcohol is prepared by reaction of the mixed anhydride with an amino alcohol (a sphingoid base is an amino alcohol) or a salt thereof,
- the ceramide derivatives of the present invention are obtained by alkaline hydrolysis of the N-2-acyloxyacylamido alcohol.

In another aspect of the invention, the AHA-based ceramides are used in the preparation of cosmetic and/or pharmaceutical compositions.

Specific cosmetic and/or pharmaceutical preparations include components known to those skilled in the art. The composition comprises a vehicle to enable the active ingredient to be conveyed to the skin. Vehicles include water, solids and liquids. These are classified as emollients, propellants, solvents, humectants, thickeners, penetration enhancers and powders.
Emollients include alkyl higher fatty acids, oils, and higher alcohols.
Propellants include propane, butane, isobutane, dimethyl ether, chlorofluoroalkanes, carbon dioxide, nitrous oxide. Solvents include ethyl alcohol, methylene chloride, isopropanol, ethyl ethers, DMSO.
Humectants include glycerin, gelatin, sorbitol.
Penetration enhancers include solvents, oils, surface active compounds.
Powders include chalk, talc, starch, gums.

The combination of the said components can account for 10 to 99% of the composition.

The compositions containing the compounds of the invention are suitable for topical use. The amount of active ingredient or mixture thereof suitable for topical application ranges from 0.001% to 10%, preferably from 0.005% to 2%, most preferably from 0.01% to 1% by weight of the composition.

Specifically, the compositions containing the compounds of the present invention can be used to improve the structure and condition of the skin. Whereby the improvement includes an increased protection of the skin against irritation, an increased protection of the skin against inflammation, a decreased water loss from the skin, a decreased irritation of the skin, an accelerated exfoliation of the skin, an increased smoothness of the skin.

By topical application of the compositions containing the compounds of the present invention, it has been found that the AHA-based ceramides have multifunctional effects.

The compounds of the present invention, on the one hand, indeed display the effects of short chain AHA's. They are shown to increase desquamation (exfoliation) of the skin, as a consequence of diminished corneocyte cohesion, and to decrease skin roughness.

On the other hand, the AHA-based ceramides of the present invention surprisingly show the effects of skin-identical ceramides, i.e. strengthening and maintaining of the lipid lamellar barrier of the stratum corneum. This is demonstrated by the ability of the AHA-based ceramides to protect the skin against irritation.

The present invention is exemplified by the preparation of several 2-hydroxycarboxylic acid-containing ceramide 6 derivatives, i.e. N-(2-hydroxypropanoyl)-, N-(2-hydroxyoctanoyl)- and N- (2-hydroxydecanoyl) -phytosphingosine.

The phytosphingosine which is used is obtainable efficiently by deacetylation of tetra-acetylphytosphingosine, which on its turn can be obtained in large amounts by microbial fermentation, especially by fermentation of Hansenula ciferri.

Cosmetic compositions comprising these compound are described, as well as topical application of these compositions.

### Experimental

### Measurement equipment

### Roughness

Roughness was measured by profilometry using a stylus instrument (OFR 01; Romano GmbH, Cologne, Germany). Silicon impressions from the test areas of the volar forearm were prepared. The dental mass Silasoft N® (Detax-K. Huber K.G., Karlsruhe, Germany) was used for making the impressions. The parameter mean depth of roughness R_{z} (DIN 4768/1) was measured. By convention the scan length was divided into three equal-sized areas. In each area the distance from the highest peak to the lowest trough was established. The average of these five distances is R_{z}.

### Trans-epidermal water loss

Measurements of trans-epidermal water loss (TEWL) were performed with the Tewameter (Courage & Khazaka, Cologne, Germany). The Tewameter is a device for measurement of water evaporation on skin surfaces based on the diffusion principle discovered by A. Fick in 1885.

### Skin colour

Skin colour was measured by chromametry with a Minolta Chromameter CR 300 (Minolta, Ahrensburg, Germany) in compliance with the Commission International de l'eclairage (CIE) system, according to which the registration of colour is adjusted to the non-linear colour sensitivity of human eye. A colour is expressed in a three-dimensional coordinate system with green-red (a*), yellow-blue (b*) and L* axes (brightness). The skin surface is illuminated by a Xenon flash light and remitted light registered and analysed by a photoreceiver. Chromametry is sensitive and accurate for the characterization of redness of skin irritation. In inflamed skin a positive change on the a* axis is observed, towards red. Each value was the average of three recordings.

### Desquamation

The stratum corneum was stained with dansyl chloride. Dansyl chloride was mixed to homogeneity at 5% (w/w) in petrolatum and applied to the volar forearms of the volunteers under semi-occlusive tape, one day before the beginning of the study. After 24 h the patches were removed and any excess material wiped off under running tape water. Fluorescence was assessed under UV illumination for intensity and homogeneity by a trained evaluator using the following scale for fluorescence extraction:
0 : no fluorenscence extraction
1 : slight fluorescence extraction
2 : moderate fluorescence extraction
3 : strong fluorescence extraction
4 : complete fluorescence extraction.
Half scores were used when needed.

### Test method

Two panels of each five female volunteers at the age of 19-55 years with healthy skin were included in the test.

Measurements were carried out at a temperature of 22±1°C and a relative humidity of 60±10%. Subjects were accustomed to ambient conditions for 20 min prior to any measurement. The test was carried out on the volar forearms. Initially untreated skin was measured in all six areas to find baseline values. Then the five test products were applied, one area remained untreated. The dose of applicaton was about 2 mg/cm². In the following 7 days a home application in the morning and evening took place. Measurements were evaluated during the treatment period on day 7 two hours after the last daily application. Then the test areas on both forearms were treated with a 5% aqueous solution of sodium lauryl sulphate (SDS) and an occlusive dressing applied to induce skin irritation. The dressing was removed two hours later, and the regions were gently washed with water and air-dried. After one hour the measurements were done when the level had stabilised.

Use of other cosmetic products was restricted on the test areas throughout the whole study.

### Time of evaluation

- before start treatment;
- two hours after last application on day 7;
- 1 hour after irritation with SDS (2 hours under occlusion).

| Formulations tested | |
|---|---|
| **Oil-in-water emulsions with ceramide 6 analogues** | |
| Ceteareth-6 (and) stearyl alcohol | 3.25 |
| Ceteareth-25 | 1.75 |
| Caprylic/capric triglyceride | 3.00 |
| Stearyl Beeswaxate | 1.00 |
| Decaglyceryl pentaisostearate | 5.00 |
| Cetyl alcohol | 2.50 |
| Avocado Oil | 5.00 |
| Octyl stearate | 2.00 |
| Dioctylcyclohexane | 3.00 |
| **Ceramide 6 analogue** | **0** |
| | **0.05** |
| | **0.20** |
| | **0.50** |
| Preservative* | 0.50 |
| Water | ad 100 |

| | |
|---|---|
| * Propylene glycol (and) phenoxyethanol (and) methylparaben (and) propylparaben (and) ethylparaben (and) butylparaben | |

### Example 1

### Synthesis of N-[(S)-2-hydroxypropanoyl]-phytosphingosine (Cer6/OH-C3)

### a. (S)-2-acetoxypropanoic acid

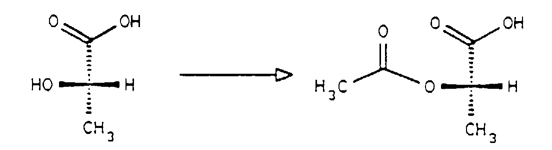

A mixture of (S)-2-hydroxypropanoic acid (120 ml; 135 g; 1.5 mole), acetic acid (500 ml), toluene (100 ml) and concentrated sulphuric acid (1 ml) was heated under reflux with a Dean Stark apparatus. During collecting the water in the trap more toluene (2 x 100 ml) and acetic acid (200 ml) was added. After the addition of sodium acetate (4 g), the reaction mixture was distilled at reduced pressure, thus yielding 99.2 g of the (S)-2-acetoxypropanoic acid at 116 - 130 °C/0.2 mm Hg.

### b. N-[(S)-2-acetoxypropanoyl]-phytosphingosine

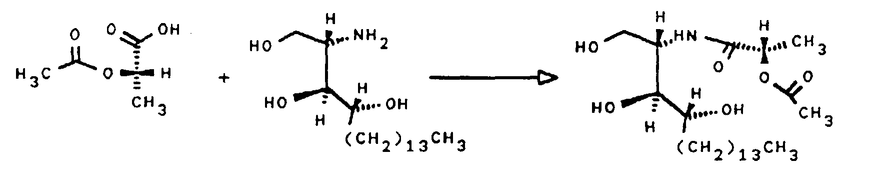

A mixture of (S)-2-acetoxypropanoic acid (24.38 g; 184 mmoles), triethyl amine (60 ml) and ethyl acetate (90 ml) was added dropwise under nitrogen to a stirred solution of tosyl chloride (32.4 g) in ethyl acetate (250 ml) at 31 °C. After stirring for 20 minutes at 45 °C, this mixture was added to a stirred suspension (39 °C) of phytosphingosine sulphate (51 g; ca 63 mmoles) in ethyl acetate (165 ml) and triethyl amine (30 ml) under nitrogen in about 10 minutes. After stirring for 2 hours at 50 °C water (150 ml) was added. The layers were separated and 150 ml of water was added to the organic layer. After adjusting the pH at 2.5 with HCl (36 %) the organic layer was separated, washed with a sodium chloride solution (100 ml; 20 %-solution) and evaporated to dryness in vacuo at a rotavapor. Then 100 ml of methanol was added and the methanol was evaporated again. After dissolving the residue in 200 ml of warm methanol the solution was cooled down to 1 °C and filtrated with an isolated filter in order to keep the temperature low. After washing with 50 ml of cold methanol and drying in vacuo 31.31 g of N-[(S)-2-acetoxypropanoyl]-phytosphingosine was obtained.
PMR-spectrum (360 MHz; DMSO-d6; values in ppm).
δ: 0.84 (t, 3H); 1.23 (22H); 1.29 (d, 3H); 1.43 (m, 4H); 2.04 (s, 3H); 3.36 (m, 2H); 3.51 (m, 2H); 3.87 (m, 1H) ; 4.30 (d, 1H) ; 4.48 (t, 1H) ; 4.62 (d, 1H) ; 4.96 (q, 1H) ; 7.60 (d, 1H).

### c. N-[(S)-2-hydroxypropanoyl]-phytosphingosine

To a stirred mixture of N-[(S)-2-acetoxypropanoyl]-phytosphingosine (10.23 g) and methanol (50 ml) a NaOH solution 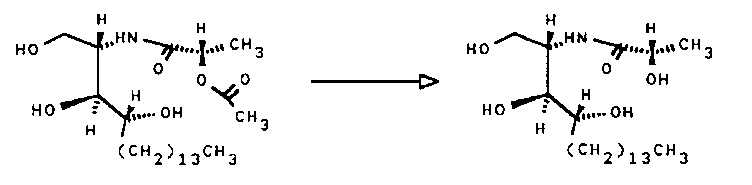 (1.23 g of NaOH in 1.23 ml of water) was added. After stirring for 1 hour at room temperature and adjusting the pH to 7 with HCl (36 %) the reaction mixture was filtrated and concentrated at the rotavapor in vacuo. The residue was stirred with water (100 ml), filtered off, washed with water and dried, yielding 8.33 g of N-[(S)-2-hydroxypropanoyl]-phytosphingosine. Purity according to NMR-assay 93 %; 4-nitrotoluene as an internal standard).
PMR-spectrum (360 MHz; DMSO-d6; values in ppm; δ DMSO: 2.49). δ: 0.84 (t, 3H) ; 1.23 (22H); 1.42 (m, 2H) ; 1.55 (m, 2H); about 3.3 (2H); 3.51 (m, 2H); 3.93 (m, 2H); 4.38 (d, 1H); 4.60 (t, 1H) ; 4.70 (d, 1H) ; 5.51 (d, 1H) ; 7.31 (d, 1H).

### Example 2

### Synthesis of N-(2-hydroxyoctanoyl)-phytosphingosine (Cer6/OH-C8)

### a. 2-Bromo-octanoic acid

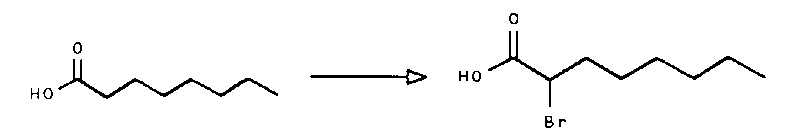

A mixture of octanoic acid (288 g; purity 99%; 2 moles) and phosphorous trichloride (10 ml) was stirred with slight suction with a waterjet pump to remove acid vapours and heated at 60°C. Next bromine (100 ml) was added over a period of 1 hour.

The mixture was stirred at 60°C for 1 day. After stirring overnight at room temperature stirring was continued at 70°C for another day. More bromine was added (25 ml) and stirring was continued overnight at 70°C. The next day more phosphorous trichloride (10 ml) and more bromine (10 ml) were added and stirring was continued. At the end of the day more bromine (15 ml) was added and stirring was continued overnight at 60°C. The conversion into 2-bromo-octanoic acid was estimated at 80% by TLC.

The mixture was heated to 110°C with suction to remove excess bromine and stirred with 1 litre of water. The organic layer was separated and stirred again with 1 litre of water. The organic layer containing the 2-bromo-octanoic acid was used as such to prepare 2-hydroxyoctanoic acid.

### b. 2-Hydroxyoctanoic acid

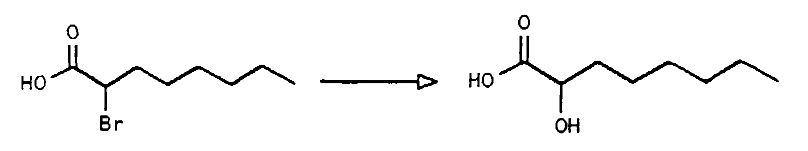

The 2-bromo-octanoic acid prepared as in example 2a was stirred and heated with a solution of 200 grams of sodium hydroxide in 2 litres of water under nitrogen at 80°C. When foaming ceased the mixture was stirred for 2 hours at 95°C, cooled to 80°C and acidified with 350 ml of 36% hydrochloric acid.

After cooling to 30°C the mixture was extracted with toluene (500 ml). The extract was evaporated to give 327 grams of an oil. This was dissolved in hot hexane (700 ml) and filtered hot. The filter was washed with 50 ml of hot hexane.
The filtrate was cooled to 7°C with stirring and the precipitate formed was filtered off, washed with cold hexane (250 and 200 ml) and dried in vacuo to give 201.24 grams of the title product.

### c. 2-Acetoxyoctanoic acid

Acetic anhydride (40 ml) was added to a stirred and ice-cooled mixture of 2-hydroxyoctanoic acid (30 g) and pyridine (80 ml). After stirring overnight at room temperature water 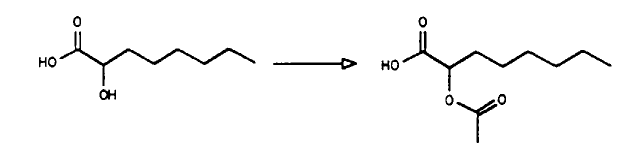 (100 ml) was added while cooling and stirring was continued for 1 hour at room temperature.

The mixture was acidified to pH=1 with 90 ml of 36% hydrochloric acid and extracted with toluene (100 ml and 50 ml).

The combined toluene extracts were washed with 50 ml of 1M HCl, with water (2 x 100 ml) and with brine.
After filtration the toluene solution was evaporated in vacuo to give 39.9 grams of the title compound.

### d. N-(2-acetoxyoctanoyl)sphingosine

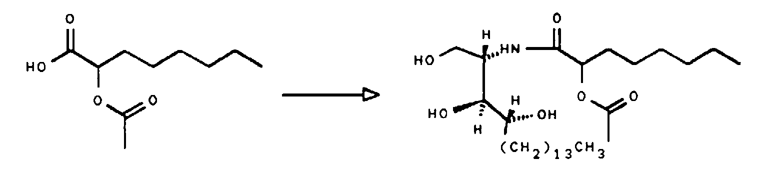

A mixture of 2-acetoxyoctanoic acid (8.80 g), dry ethyl acetate (25 ml) and triethyl amine (13 ml) was added to a stirred solution of 7 grams of p-toluene-sulphonyl chloride in 50 ml of ethyl acetate during 10 minutes at 45°C. After stirring for 30 minutes the suspension was added in 5 minutes to a stirred suspension of phytosphingosine sulphate (12 g) in ethyl acetate (50 ml) and triethyl amine (6.5 ml). Stirring was continued for 1 hour at 46°C and 100 ml of water were added. The pH was adjusted to 6.2 with 36% hydrochloric acid and the organic layer was separated and washed with 100 ml of water and evaporated to give an oil. This was treated with 25 ml of methanol. After evaporation of the methanol 18.57 grams of a solid was obtained. Of this material 1.06 grams were dried in vacuo to give 0.98 g of the dried title compound.

### e. N-(2-hydroxyoctanoyl)-phytosphingosine

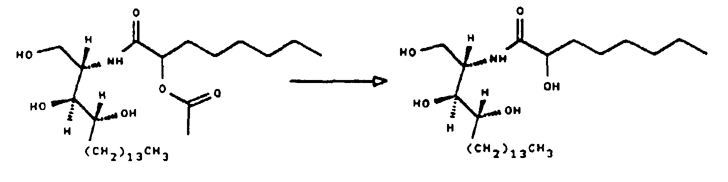

A mixture of N-2-(R,S)-acetoxyoctanoyl-phytosphingosine (26 g) and methanol (80 ml) was stirred. Next a freshly prepared solution of 1.6 g of sodium hydroxide in 1.6 grams of water was added and the mixture was stirred at room temperature for 3 hours.

After neutralisation with 1 ml of acetic acid the mixture was filtered (filter washed with 30 ml of methanol). Next 80 ml of water were added and stirring was continued for 5 minutes.

The precipitate was filtered off, washed with 50 ml of methanol/water=1/1 and 2x 100 ml of water and dried in vacuo to give 22.01 g of the title compound.
PMR-spectrum (360 MHz; CDCl₃-d6, pyridin-d5, 2 dr. of DCOOD; T: 323 °K; values in ppm).
δ: 0.92 (t, 6H); 1.3-2.0 (m, 36H); 3.95 (m, 2H); 4.1-4.2 (m, 2H); 4.35 (m, 1H) ; 4.64 (m, 1H); 8.13 (2xd, 1H).

### Example 3

### Synthesis of N-(2-hydroxydecanoyl)-phytosphingosine

### a. 2-Bromodecanoic acid

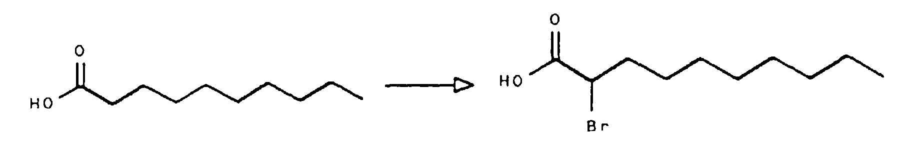

A mixture of decanoic acid (430 g; 2.5 moles) and phosphorus trichloride (13 ml; 0.15 mole) was heated at 60°C. Next bromine (155 ml; 3.0 moles) was added in 10 minutes while keeping the temperature at 60 °C and keeping a slight vacuum with a water jet pump to remove HBr fumes.

Stirring was continued overnight at 60 °C and at 90 °C until the reaction mixture decolourized. Next the mixture was cooled down, diluted with 1 l of toluene and washed with water (3 x 0.25 1 and 2 x 0.25 1 at pH=2). In order to give a good separation some diethyl ether (1 1) was added. After the organic layer had been dried with sodium sulphite it was concentrated at diminished pressure (0.5 mm Hg) at 90 °C, giving 595 grams of the title compound.

### b. 2-Hydroxydecanoic acid

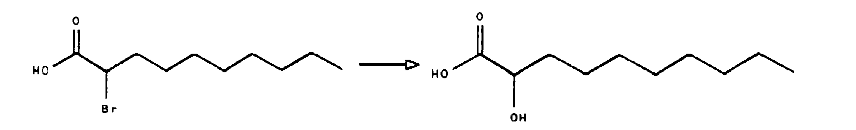

A mixture of 2-bromodecanoic acid (prepared from 175 grams of decanoic acid) and a solution of 80 grams of sodium hydroxide in 1 litre of water was stirred under nitrogen at 85 to 90 °C. More sodium hydroxide (25 g) was added and stirring was continued for 2.5 hours.

Next the mixture was acidified with 125 ml of 36% hydrochloric acid and extracted with 450 ml of toluene. The extract was evaporated to give 210 g of an oil. After dilution with 900 ml of hexane crystallisation started. After stirring for 0.5 hours the precipitate was filtered off, washed with 250 ml of hexane and dried to give 101.10 grams of the title product.

### c. 2-Acetoxydecanoic acid

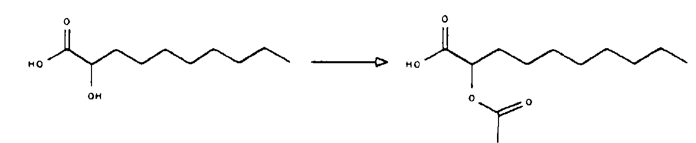

Acetic anhydride (20 ml) was added slowly to a stirred mixture of 2-hydroxydecanoic acid (15.34 g) and pyridine (40 ml) while cooling in a water bath at room temperature. After stirring for 2 hours 50 ml of water was added and the pH was adjusted to 2 with 36% hydrochloric acid. The mixture was extracted with toluene and the extract was washed with diluted hydrochloric acid and concentrated to give 19.39 grams of the title compound.

### d. N-(2-acetoxydecanoyl)-phytosphingosine

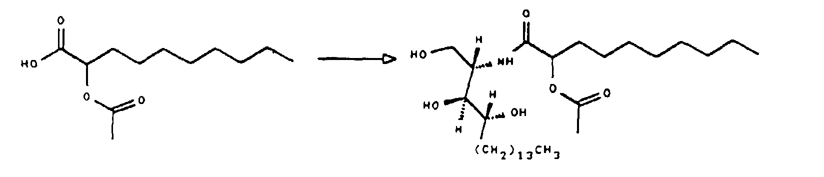

A mixture of 2-Acetoxydecanoic acid (19.0 g), dry ethyl acetate (45 ml) and triethyl amine (24 ml) was added to a stirred solution of p-toluene-sulphonyl chloride (13 g) in ethyl acetate (90 ml) at 45°C during 20 minutes . After stirring for 0.5 hours the suspension was added in 10 minutes to a stirred suspension of phytosphingosine sulphate (19 g) in ethyl acetate (100 ml) and triethyl amine (12 ml). Stirring was continued for 1 hour at 46°C and 200 ml of water were added. After the pH was adjusted to acidic with 9 ml of 36% hydrochloric acid the organic layer was separated, washed with 100 ml of water and evaporated to give an oil. This was treated with 100 ml of methanol and evaporated. The residue was treated with 50 ml of methanol and the solids were filtered off. The filter cake was stirred with 100 ml of methanol, filtered off again and dried in vacuo to give 16.66 g of the title compound.

### e. N-(2-hydroxydecanoyl)-phytosphingosine

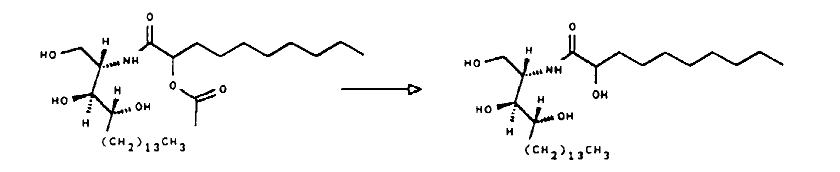

A mixture of N-(2-acetoxydecanoyl)-phytosphingosine (16 g) and methanol (80 ml) was heated to give a solution and cooled to room temperature. Next a freshly prepared solution of 1.6 grams of sodium hydroxide in 1.6 grams of water was added and the mixture was stirred at room temperature for 2.5 hours. After neutralisation with 1.5 ml of acetic acid 100 ml of water were added in portions while stirring. The precipitate was filtered off, washed with 50 ml of methanol/water=1/1 and 2x 100 ml of water and dried in vacuo to give 13.41 grams of the title compound.
PMR-spectrum (360 MHz; CDCl₃-d6, pyridin-d5, 2 dr. of DCOOD; T: 323 °K; values in ppm).
δ: 0.92 (t, 6H); 1.3-2.0 (m, 36H); 3.95 (m, 2H); 4.1-4.2 (m, 2H); 4.35 (m, 1H) ; 4.64 (m, 1H) ; 8.13 (2xd, 1H).

### Example 4

### Smoothening effect of Ceramide 6 analogues on healthy human skin as measured with profilometry

Skin roughness measurements have been performed with the ceramide 6 analogues Cer6/OH-C3 and Cer6/OH-C8. The results of these measurements are indicated in Tables 1 and 2. The day 7 and SDS values should be compared to the corresponding starting value of day 0.

A decrease in roughness, much higher than the placebo, was found after 7 days in the areas treated with the formulations containing the ceramide 6 analogues. After irritation with SDS, the roughness increased in the untreated area and in the area pretreated with placebo. Only minimal changes were detected in the areas pretreated with the ceramide containing formulations.

**Table 1.**

| Skin roughness measurements using Cer6/OH-C3 | | | | | |
|---|---|---|---|---|---|
| | Cer6/OH-C3 | | | Placebo | Untreated |
| | 0.05% | 0.2% | 0.5% | | |
| day 0 | 131.2 ± 15.5 | 104.7 ± 9 | 131.1 ± 15.2 | 114.4 ± 8.7 | 106 ± 9.4 |
| after 7 days | 109.1 ± 16.4 | 91.9 ± 9.9 | 112.9 ± 15.2 | 106.7 ± 10.6 | 106.2 ± 9.6 |
| after SDS | 96.9 ± 8.1 | 92.9 ± 9.7 | 116.5 ± 14.8 | 119 ± 12.3 | 121.8 ± 12.8 |

**Table 2.**

| Skin roughness measurements using Cer6/OH-C8 | | | | | |
|---|---|---|---|---|---|
| | Cer6/OH-C8 | | | Placebo | Untreated |
| | 0.05% | 0.2% | 0.5% | | |
| day 0 | 133.6 ± 12.5 | 147.1 ± 15.5 | 111.2 ± 6 | 114.4 ± 8.7 | 101 ± 9.8 |
| after 7 days | 119.1 ± 8.8 | 118.5 ± 8.2 | 98 ± 6.9 | 106.7 ± 10.6 | 98.2 ± 10.4 |
| after SDS | 121.8 ± 11.6 | 118.7 ± 7.6 | 82.2 ± 7.1 | 119 ± 12.3 | 119.3 ± 14 |

### Example 5

### Strengthening of the barrier function of the skin by ceramide 6 analoques as measured by TEWL

TEWL measurements have been performed using formulations containing the ceramide 6 analogues Cer6/OH-C3 and Cer6/OH-C8. The results of these measurements are presented in Tables 3 and 4. The day 7 and SDS values should be compared to the corresponding starting value of day 0.

After irritation with SDS, the increase of the TEWL in the areas pretreated with ceramide 6 analogues for 7 days was much lower compared to control and placebo pretreatment. The effect was dose dependent. This shows that pretreatment with AHA-based ceramide 6 analogues protects the skin from SDS-damage. The pretreatment itself did not show marked differences in TEWL values after 7 days compared with day 0.

**Table 3.**

| TEWL measurements using Cer6/OH-C3 | | | | | |
|---|---|---|---|---|---|
| | Cer 6, OH-C3 | | | Placebo | Untreated |
| | 0.05% | 0.2% | 0.5% | | |
| day 0 | 5 ± 1.1 | 6.1 ± 1.5 | 6 ± 1.2 | 5.8 ± 1.5 | 5.3 ± 0.5 |
| after 7 days | 8.2 ± 3.5 | 7.2 ± 1.6 | 8 ± 1.9 | 5.4 ± 2.5 | 8.2 ± 3.8 |
| after SDS | 16.6 ± 5.2 | 13.5 ± 6 | 11.6 ± 3.3 | 18.1 ± 4.6 | 32.6 ± 22.7 |

**Table 4.**

| TEWL measurements using Cer6/OH-C8 | | | | | |
|---|---|---|---|---|---|
| | Cer 6, OH-C8 | | | Placebo | Untreated |
| | 0.05% | 0.2% | 0.5% | | |
| day 0 | 5.7 ± 1.3 | 5.4 ± 0.7 | 6.5 ± 2.2 | 5.8 ± 1.5 | 5.5 ± 0.5 |
| after 7 days | 5.7 ± 1.9 | 5.5 ± 1.6 | 7.9 ± 2.7 | 5.4 ± 2.5 | 4.6 ± 2 |
| after SDS | 14.7 ± 4.2 | 10.12 ± 1.9 | 13 ± 4.4 | 18.1 ± 4.6 | 22 ± 7.7 |

### Example 6

### Strengthening of the barrier function of the skin by ceramide 6 analogues as measured with chromametry

Skin colour measurements have been performed using formulations containing the ceramide 6 analogues Cer6/OH-C3 and Cer6/OH-C8. The results of these measurements are presented in Tables 5 and 6. The day 7 and SDS values should be compared to the corresponding starting value of day 0.

After irritation with SDS, the increase of the skin colour in the areas pretreated with Ceramide 6 analogues for 7 days was much lower compared to control and placebo. The effect was dose dependent. This shows that pretreatment with AHA-based ceramide 6 analogues protects the skin from SDS-damage. The pretreatment itself did not show marked differences in skin colour values after 7 days compared with day 0.

**Table 5.**

| Results of skin colour measurements | | | | | |
|---|---|---|---|---|---|
| | Cer 6, OH-C3 | | | Placebo | Untreated |
| | 0.05% | 0.2% | 0.5% | | |
| day 0 | 6.7 ± 0.4 | 7.8 ± 0.9 | 8.2 ± 0.5 | 6.9 ± 2.2 | 6.9 ± 0.9 |
| after 7 days | 7.6 ± 0.8 | 8.5 ± 0.9 | 8.7 ± 0.7 | 6.3 ± 1.1 | 7.2 ± 1.1 |
| after SDS | 13.1 ± 1.8 | 11.4 ± 0.6 | 9.5 ± 1.3 | 13.5 ± 0.7 | 16.2 ± 2.2 |

**Table 6.**

| Results of skin colour measurements | | | | | |
|---|---|---|---|---|---|
| | Cer 6, OH-C8 | | | Placebo | Untreated |
| | 0.05% | 0.2% | 0.5% | | |
| day 0 | 7 ± 1.2 | 6.8 ± 0.5 | 6.5 ± 0.6 | 6.9 ± 2.2 | 6.5 ± 1.7 |
| after 7 days | 6.2 ± 1.2 | 6.3 ± 0.7 | 8.3 ± 1.1 | 6.3 ± 1.1 | 6.6 ± 1.7 |
| after SDS | 9.2 ± 0.8 | 8.4 ± 0.5 | 10.2 ± 0.9 | 13.5 ± 0.7 | 16.9 ± 2.7 |

### Example 7

### Effect of Ceramide 6 analogues on healthy human skin as measured by desquamation

Desquamation measurements have been performed using formulations containing the ceramide 6 analogues Cer6/OH-C3 and Cer6/OH-C8. The results of these measurements are presented in Tables 7 and 8. The values measured on day 7 and after SDS treatment should be compared to the corresponding starting value of day 0.

The area treated with the ceramide 6 analogues showed increased desquamation after 7 days as compared to placebo and untreated area. Moreover, after treatment with SDS, the skin pretreated with ceramide 6 analogues gave much lower fluorescence extraction values than untreated and placebo pretreated skin. This shows the protecting effect of the ceramide 6 analogues on human skin against SDS-challenge.

**Table 7.**

| Desquamation measurements using Cer6/OH-C3 | | | | | |
|---|---|---|---|---|---|
| | Cer6/OH-C3 | | | Placebo | Untreated |
| | 0.05% | 0.2% | 0.5% | | |
| day 0 | 0 | 0 | 0 | 0 | 0 |
| after 7 days | 0,2 ± 0,4 | 0,2 ± 0,4 | 0,2 ± 0.4 | 0 | 0 |
| after SDS | 0,8 ± 0,4 | 1 ± 0 | 1 ± 0 | 1,6 ± 0 | 2,8 ± 0,4 |

**Table 8.**

| Desquamation measurements using Cer6/OH-C8 | | | | | |
|---|---|---|---|---|---|
| | Cer6/OH-C8 | | | Placebo | Untreated |
| | 0.05% | 0.2% | 0.5% | | |
| day 0 | 0 | 0 | 0 | 0 | 0 |
| after 7 days | 0,4 ± 0,5 | 0,4 ± 0.5 | 0,4 ± 0,5 | 0 | 0 |
| after SDS | 0.8 ± 0.4 | 0,8 ± 0,4 | 1 ± 0 | 1,6 ± 0,5 | 2,6 ± 0,5 |

## Claims

1. A compound which is a sphingoid base selected from the group consisting of sphingosine and phytosphingosine linked through an amide to a 2-hydroxycarboxylic group having a chain length of 2 to 14 carbon atoms, with the proviso that said compound is not hydroxyacetylsphingosine, 2-hydroxybutyroylsphingosine or 2-hydroxydecanoylsphinganine.

2. A compound according to claim 1 in which the 2-hydroxycarboxylic group is a 2-hydroxyethanoyl, a 2-hydroxypropanoyl, a 2-methyl 2-hydroxypropanoyl, a 2-hydroxybutanoyl, a 2-hydroxypentanoyl, a 2-hydroxyhexanoyl, a 2-hydroxyheptanoyl, a 2-hydroxyoctanoyl, a 2-hydroxynonanoyl, a 2-hydroxydecanoyl, a 2-hydroxyundecanoyl, a 2-hydroxydodecanoyl, a 2-hydroxytetradecanoyl group.

3. A compound according to claim 1 in which the 2-hydroxycarboxylic group is a 2-hydroxyethanoyl, a 2-hydroxypropanoyl, a 2-hydroxyoctanoyl or a 2-hydroxydecanoyl group.

4. A method for preparing a compound according to any one of the claims 1 to 3 comprising the following steps:
- a carboxylic acid with a chain length from 2 to 14 carbon atoms is brominated on the 2-position,
- the 2-bromocarboxylic acid is converted into a 2-hydroxy acid,
- the 2-hydroxy acid is acylated,
- the 2-acyloxycarboxylic acid is converted into a mixed anhydride with an alkyl sulfonyl chloride or an alkylaryl sulfonyl chloride, by carrying out the reaction in an organic solvent in the presence of a base,
- the mixed anhydride is reacted with an amino alcohol or a salt thereof,
- the N-2-acyloxyacylamido alcohol is hydrolyzed by alkaline hydrolysis.

5. A cosmetic or pharmaceutical composition comprising a compound according to any one of the claims 1 to 3.

6. A composition according to claim 5 comprising the compound in a concentration ranging from 0.001% to 10%, preferably from 0.005% to 2%, most preferably from 0.01% to 1% by weight of the composition.

7. A compound according to any one of the claims 1 to 3 for use in therapy.

8. Use of a compound according to any one of the claims 1 to 3 as a cosmetic.

9. Use according to claim 8 to improve the structure and condition of the skin.

## Patentansprüche

1. Verbindung, die eine Sphingoidbase ist, ausgewählt aus der Gruppe, die aus Sphingosin und Phytosphingosin besteht, das über ein Amid mit einer 2-Hydroxycarboxylgruppe mit einer Kettenlänge von 2 bis 14 Kohlenstoffatomen verknüpft ist, mit der Maßgabe, daß die genannte Verbindung nicht Hydroxyacetylsphingosin, 2-Hydroxybutyroylsphingosin oder 2-Hydroxydecanoylsphinganin ist.

2. Verbindung nach Anspruch 1, worin die 2-Hydroxycarboxylgruppe eine 2-Hydroxyethanoyl-, eine 2-Hydroxypropanoyl-, eine 2-Methyl-2-hydroxypropanoyl-, eine 2-Hydroxybutanoyl-, eine 2-Hydroxypentanoyl-, eine 2-Hydroxyhexanoyl-, eine 2-Hydroxyheptanoyl-, eine 2-Hydroxyoctanoyl-, eine 2-Hydroxynonanoyl-, eine 2-Hydroxydecanoyl-, eine 2-Hydroxyundecanoyl-, eine 2-Hydroxydodecanoyl- oder eine 2-Hydroxytetradecanoylgruppe ist.

3. Verbindung nach Anspruch 1, worin die 2-Hydroxycarboxylgruppe eine 2-Hydroxyethanoyl-, eine 2-Hydroxypropanoyl-, eine 2-Hydroxyoctanoyl- oder eine 2-Hydroxydecanoylgruppe ist.

4. Verfahren zum Herstellen einer Verbindung nach einem der Ansprüche 1 bis 3, das die folgenden Stufen umfaßt:
- Eine Carbonsäure mit einer Kettenlänge von 2 bis 14 Kohlenstoffatomen wird in der 2-Stellung bromiert,
- die 2-Bromcarbonsäure wird in eine 2-Hydroxysäure umgewandelt,
- die 2-Hydroxysäure wird acyliert,
- die 2-Acyloxycarbonsäure wird mit einem Alkylsulfonylchlorid oder einem Alkylarylsulfonylchlorid unter Durchführen der Reaktion in einem organischem Lösungsmittel in Gegenwart einer Base in ein gemischtes Anhydrid überführt,
- das gemischte Anhydrid wird mit einem Aminoalkohol oder einem Salz hiervon umgesetzt,
- der N-2-Acyloxyacylamidoalkohol wird durch alkalische Hydrolyse hydrolysiert.

5. Kosmetische oder pharmazeutische Zusammensetzung, enthaltend eine Verbindung nach einem der Ansprüche 1 bis 3.

6. Zusammensetzung nach Anspruch 5, enthaltend die Verbindung mit einer Konzentration im Bereich von 0,001 bis 10 %, vorzugsweise von 0,005 bis 2 %, insbesondere von 0,01 bis 1 %, jeweils bezogen auf das Gewicht der Zusammensetzung.

7. Verbindung nach einem der Ansprüche 1 bis 3 zur Verwendung in der Therapie.

8. Verwendung einer Verbindung nach einem der Ansprüche 1 bis 3 als Kosmetikum.

9. Verwendung nach Anspruch 8 zum Verbessern der Struktur und des Zustands der Haut.

## Revendications

1. Composé constitué par une base sphingoïde choisie dans le groupe constitué par la sphingosine et la phytosphingosine liées par une liaison amide à un groupe 2-hydroxycarboxylique dont la chaîne a une longueur de 2 à 14 atomes de carbone, sous réserve que ledit composé ne soit pas l'hydroxyacétylsphingosine, la 2-hydroxybutyroylsphingosine ou la 2-hydroxydécanoylsphinganine,

2. Composé selon la revendication 1, dans lequel le groupe 2-hydroxycarboxylique est un groupe 2-hydroxyéthanoyle, 2-hydroxypropanoyle, 2-méthyl 2-hydroxypropanoyle, 2-hydroxybutanoyle, 2-hydroxypentanoyle, 2-hydroxyhexanoyle, 2-hydroxyheptanoyle, 2-hydroxyoctanoyle, 2-hydroxynonanoyle, 2-hydroxydécanoyle, 2-hydroxyundécanoyle, 2-hydroxydodécanoyle, 2-hydroxytétradécanoyle.

3. Composé selon la revendication 1, dans lequel le groupe 2-hydroxycarboxylique est un groupe 2-hydroxyéthanoyle, 2-hydroxypropanoyle, 2-hydroxyoctanoyle ou 2-hydroxydécanoyle.

4. Méthode pour la préparation d'un composé selon une quelconque des revendications 1 à 3 comprenant les étapes suivantes :
- on brome en position 2 un acidc carboxylique dont la chaîne présente une longueur de 2 à 14 atomes de carbone,
- on convertit l'acide 2-bromocarboxylique en acide 2-hydroxy,
- on acyle l'acide 2-hydroxy,
- on convertit l'acide 2-acyloxycarboxylique en un anhydride mixte avec un chlorure d'alkylsulfonyle ou un chlorure d'alkylarylsulfonyle par réaction dans un solvant organique en présence d'une base,
- on fait réagir l'anhydride mixte avec un aminoalcool ou un de ses sels,
- on hydrolyse le N-2-acyloxyacylamidoalcool par hydrolyse alcaline.

5. Composition cosmétique ou pharmaceutique comprenant un composé selon une quelconque des revendications 1 à 3.

6. Composition selon la revendication 5 comprenant le composé en une concentration allant de 0,001 % à 10 %, de préférence de 0,005 % à 2 %, plus preférablement de 0,01 % à 1 % en masse par rapport à la composition.

7. Composé selon une quelconque des revendications 1 à 3 pour son utilisation en thérapeutique.

8. Utilisation d'un composé selon une quelconque des revendications 1 à 3 en tant que cosmétique.

9. Utilisation selon la revendication 8, pour améliorer la structure et l'état de la peau.
